# EUROPÄISCHE PATENTSCHRIFT

(11) **EP 0 523 412 B1**
(45) Veröffentlichungstag und Bekanntmachung des Hinweises auf die Patenterteilung: **25.10.1995**
(21) Anmeldenummer: 92110623.3
(22) Anmeldetag: 24.06.1992
(51) Int. Cl.: A61B 17/60

(54) **Klemmkupplung zum Verbinden von Knochenschrauben mit einem äusseren Fixateur**
Clamping joint for connecting bone screws to an external fixator
Accouplement à serrage pour rattacher des vis à os à un fixateur externe

(30) Priorität: 16.07.1991 DE 4123439
(43) Veröffentlichungstag der Anmeldung: 20.01.1993
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.

(56) Entgegenhaltungen:
- EP-A- 0 011 258
- EP-A- 0 140 591
- JOURNAL OF BONE AND JOINT SURGERY. Bd. 72-A, Nr. 4, April 1990, BOSTON US Seiten 601 - 618 B.F.MORREY 'Post-Traumatic Contracture of the Elbow'

## Beschreibung

Die Erfindung bezieht sich auf eine Klemmkupplung zum Festlegen von Knochenschrauben oder -pins und zum Verbinden derselben mit einem äußeren Fixateur gemäß dem Oberbegriff des Hauptanspruches.

Ein äußerer Fixateur, der aus einer Spannvorrichtung und zwei an diese Spannvorrichtung anschließenden Klemmkupplungen besteht, ist aus der US 43 12 336 bekannt. Die Klemmkupplungen bestehen dabei aus zwei Halbschalen, die aufeinander festgelegt werden können und zwischen sieh die Knochenschrauben oder Pins od. dgl. einklemmen können. Diese Kupplungen schließen dann über einen Kugelgelenkanschluß in Form eines Bajonettverschlusses an die eigentliche Spannvorrichtung des Fixateurs an.

Bei bis in das Gelenk eines Oberschenkelknochens reichenden Brüchen besteht die Schwierigkeit, diese bekannten Klemmkupplungen mit ausreichender Sieherheit festzulegen.

Der Erfindung liegt daher die Aufgabe zugrunde, eine bessere Verankerung bei bis in das Gelenk reichenden Brüchen für einen zum Stand der Technik gehörenden Fixateur zu schaffen.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt wird vorgeschlagen, daß an die an sich bekannte und auch im vorliegenden Fall zum Einsatz kommende Klemmkupplung eine zweite Klemmkupplung angeschlossen wird, die aber quer zur ersten Klemmkupplung ausgerichtet ist, d. h. die beiden Längsachsen der Klemmkupplungen stehen senkrecht aufeinander. Die Verbindung der beiden Klemmkupplungsteile, d. h. der eigentlichen Klemmkupplung und der Querklemmkupplung, erfolgt dadurch, daß an der Klemmkupplung an der den Kugelgelenkanschluß abgewandten Steite ein Verbindungszapfen vorgesehen ist, der in eine Aufnahmebohrung der Halbschale oder des Hauptkörpers der Querklemmkupplung reicht. Hier kann dieser Verbindungszapfen dann über eine entsprechende Festlegvorrichtung an der Halbschale oder dem Hauptkörper verankert werden, wobei wesentlich ist, daß der Mittelpunkt der Aufnahmebohrung auf der Längsachse der Halbschale seitlich versetzt ist. Dieser seitliche Versatz trägt der Schaftmittellinie des Oberschenkelknochens in der seitlichen Ansicht Rechnung, und vorzugsweise beträgt dieser Versatz etwa 7 mm.

Ausführungsbeispiele der Erfindung werden nachfolgend anhand der Zeichnungen erläutert. Die Zeichnungen zeigen dabei in
- Fig. 1: in einer auseinandergezogenen Darstellungsweise die verschiedenen Bauteile der neuen Kupplung, in
- Fig. 2: zur Verdeutlichung des Versatzes eine rein schematische Ansicht eines Condylus mit eingezeichneter Schaftmittellinie und eingezeichneter Mittellinie des Gelenkkopfes und in
- Fig. 3: eine abgeänderte Ausführungsform der Querklemmkupplung.

In der Zeichnung ist eine Klemmkupplung 1 dargestellt, die einen Kugelgelenkanschluß 6 für einen Bajonettverschluß aufweist und im wesentlichen aus zwei Halbschalen 2 und 3 besteht, die über Schrauben 4 aufeinander und aneinander festgelegt werden können. Die Halbschalen 2 und 3 sind auf ihren aufeinanderzu gerichteten Seiten mit halbkreisförmigen Aufnahmen 5 ausgerüstet, in die Knochenschrauben oder Knochenpins eingesetzt werden können, wobei diese Knochenschrauben od. dgl. dann durch aufeinander Festlegen der Halbschale 2 auf der Halbschale 3 ebenfalls festgelegt werden. Ober ein solches Hilfsmittel erfolgt also die Verbindung zwischen dem Knochen über die Knochenschraube zum eigentlichen Fixateur.

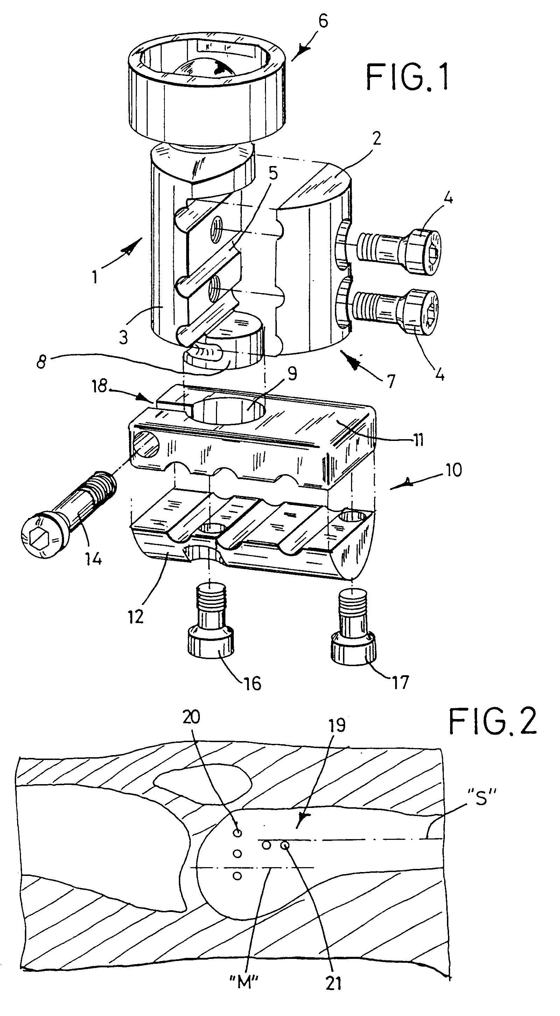

Bei dem dargestellten Ausführungsbeispiel gemäß Fig. 1 schließt an eine dem Kugelgelenkanschluß 6 gegenüberliegende Stirnseite 7 der Klemmkupplung 1 eine Querklemmkupplung 10 an, die ebenfalls aus zwei Halbschalen 11 und 12 besteht, die mit Aufnahmen für Knochenschrauben od. dgl . ausgerüstet sind. Auch in diesem Fall werden die Halbschalen 11 und 12 durch entsprechende Schrauben 16 und 17 aneinander festgelegt.

Die Stirnseite 7 der Klemmkupplung 1 und dort der Halbschale 3 trägt einen Verbindungszapfen 8, der in eine in der Halbschale 11 der Querklemmkupplung 10 vorgesehene Aufnahmebohrung 9 eingesetzt werden kann. Die Festlegung erfolgt durch eine Spannschraube 14 und dadurch, daß ein Teil der Halbschale, beispielsweise reichend bis zur Aufnahmebohrung 9, geschlitzt ist, wie dies deutlich bei 18 erkennbar ist.

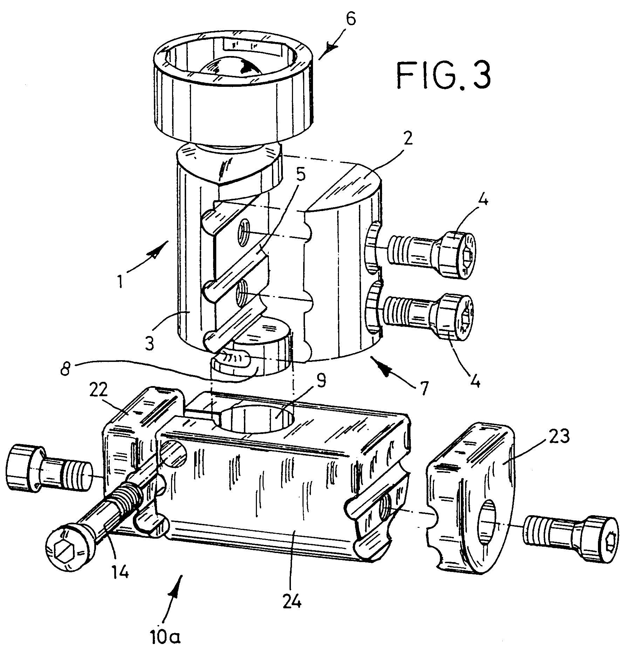

Bei der Ausführungsform gemäß Fig. 3 besteht eine Querklemmkupplung 10a aus einem Hauptkörper 24 und zwei Halbschalen 22 und 23, die übereinanderliegende Aufnahmen für Knochenpins oder Knochenschrauben aufweisen.

Aus der Zeichnung ebenfalls deutlich zu erkennen ist die Tatsache, daß das Zentrum der Aufnahmebohrung 9 auf der Längsachse der Halbschale 11 oder der Hauptkörper 24 außermittig verschoben ist, wobei dieses außermittige Verschieben etwa 7 mm beträgt. Hierdurch wird ein seitlicher Versatz zwischen der Querklemmkupplung 10 oder 10a und der Klemmkupplung 1 erreicht, der dem seitlichen Versatz der Schaftmittellinie des Oberschenkelknochens Rechnung trägt, und zwar in seitlicher Ansicht. Dies wird deutlich aus der Darstellung in Fig. 2. Hier ist ein Condylus 19 dargestellt, und bei "S" ist die Schaftmittellinie des Oberschenkelknochens eingezeichnet, während die Mittellinie des Gelenkkopfes mit "M" bezeichnet ist. Es ist ersichtlich, daß diese beiden Mittelinien gegeneinander versetzt sind, und bei 20 sind die Bohrungen zur Aufnahme der von der Querklemmkupplung 10 oder 10a getragenen Knochenschrauben oder Knochenpins erkennbar, während bei 21 die von der normalen Klemmkupplung 10 getragenen Bohrungen eingezeichnet sind. Auch aus dieser Darstellung ist ersichtlich, wie diese beiden Bohrungen hinsichtlich ihrer Mittellinie oder Mittelachse gegeneinander versetzt sind.

Mit anderen Worten ausgedrückt, wird durch die vorbeschriebene außermittige Anordnung des Anschlußpunktes, d. h. des Verbindungszapfens 8 eine parallele Einstellung der Befestigungsvorrichtung zur Längsachse des Knochens erreicht.

## Patentansprüche

1. Klemmkupplung (1) zum Festlegen von Knochenschrauben oder -pins und zum Verbinden derselben mit einem äußeren Fixateur, bestehend aus zwei mittels Schrauben (4) verbindbaren, und Aufnahmen (5) für die Knochenschrauben oder -pins aufweisenden Halbschalen (2, 3) und einem Kugelgelenkanschluß (6), gekennzeichnet durch eine an der Klemmkupplung (1) festlegbare, mit ihrer Längsachse senkrecht zur Längsachse der Klemmkupplung (1) ausgerichtete Querklemmkupplung (10), die mit Halbschalen (11, 12) zur Festlegung von weiteren Knochenschrauben oder -pins ausgebildet ist.

2. Klemmkupplung nach Anspruch 1, dadurch gekennzeichnet, daß eine Querklemmkupplung (10a) vorgesehen ist, die aus einem Hauptkörper (24) und zwei Halbschalen (22, 23) gebildet ist.

3. Klemmkupplung nach Anspruch 1 oder 2, gekennzeichnet durch einen an der dem Kugelgelenkanschluß (6) gegenüberliegenden Stirnwand (7) der Klemmkupplung (1) vorgesehenen Verbindungszapfen (8), der mit einer Aufnahmebohrung (9) in der Halbschale (11) der Querklemmkupplung (10) bzw. einem Hauptkörper (24) der Querklemmkupplung (10a) zusammenwirkt und damit die Querklemmkupplung (10, 10a) an der Klemmkupplung (1) festlegt.

4. Klemmkupplung nach Anspruch 1, 2 oder 3, dadurch gekennzeichnet, daß der Mittelpunkt der Aufnahmebohrung (9) auf der Längsachse der Halbschale (11) bzw. des Hauptkörpers (24) seitlich versetzt ist.

## Claims

1. A compression coupling (1) for fixing bone screws or pins and connecting them with an external fixer, consisting of two half-shells (2, 3), connectable by means of screws (4) and comprising receptacles (5) for the bone screws or pins, and of a ball-and-socket joint connection (6), characterized by a transverse compression coupling (10) fastenable to the compression coupling (1) and oriented with its longitudinal axis perpendicular to the longitudinal axis of the compression coupling (1), which transverse compression coupling (10) is constructed with half-shells (11, 12) for fixing further bone screws or pins.

2. A compression coupling according to claim 1, characterized in that a transverse compression coupling (10a) is provided which is constructed of a main member (24) and two half-shells (22, 23).

3. A compression coupling according to claim 1 or claim 2, characterized by a connecting peg (8) provided on the end wall (7), opposite the ball-and-socket joint connection (6), of the compression coupling (1), which connecting peg (8) interacts with a receiving bore (9) in the half-shell (11) of the transverse compression coupling (10) or a main member (24) of the transverse compression coupling (10a) and thus fixes the transverse compression coupling (10, 10a) to the compression coupling (1).

4. A compression coupling according to claim 1, 2 or 3, characterized in that the centre point of the receiving bore (9) is laterally displaced on the longitudinal axis of the half-shell (11) or the main member (24).

## Revendications

1. Accouplement à serrage (1) pour fixer des vis ou broches à os et relier celles-ci à un fixateur externe, composé de deux demi-coques (2, 3) pouvant être reliées au moyen de vis (4) et présentant des logements (5) pour les vis ou broches à os ainsi qu'un joint à rotule (6), caractérisé en ce qu'il comporte un accouplement à serrage transversal (10) qui peut être fixé sur l'accouplement à serrage (1) et est orienté sur son axe longitudinal perpendiculairement à l'axe longitudinal de l'accouplement à serrage (1), et qui est pourvu de demi-coques (11, 12) en vue de la fixation d'autres vis ou broches à os.

2. Accouplement à serrage selon la revendication 1, caractérisé en ce qu'il est prévu un accouplement à serrage (10a), qui est formé d'un corps principal (24) et de deux demi-coques (22, 23).

3. Accouplement à serrage selon la revendication 1 ou 2, caractérisé en ce qu'il comporte une cheville de liaison (8), prévue sur la paroi frontale (7) de l'accouplement à serrage (1) opposée au joint à rotule (6), qui coopère avec un trou de réception (9) dans la demi-coque (11) de l'accouplement à serrage transversal (10) ou un corps principal (24) de l'accouplement à serrage transversal (10a) et fixe ainsi l'accouplement à serrage transversal (10, 10a) sur l'accouplement à serrage (1).

4. Accouplement à serrage selon la revendication 1, 2 ou 3, caractérisé en ce que le centre du trou de réception (9) est décalé latéralement sur l'axe longitudinal de la demi-coque (11) ou du corps principal (24).
